(19) Europäisches Patentamt
European Patent Office
Office européen des brevets

(11) Veröffentlichungsnummer: **0 564 805 A1**

# (12) EUROPÄISCHE PATENTANMELDUNG

(21) Anmeldenummer: **93103179.3**

(51) Int. Cl.[5]: **C07D 237/32**, A61K 35/50

(22) Anmeldetag: **27.02.93**

(30) Priorität: **07.03.92 DE 4207234**

(43) Veröffentlichungstag der Anmeldung:
**13.10.93 Patentblatt 93/41**

(84) Benannte Vertragsstaaten:
**AT BE CH DE DK ES FR GB GR IE IT LI LU MC NL PT SE**

(71) Anmelder: **ASTA Medica Aktiengesellschaft**
**An der Pikardie 10**
**D-01277 Dresden(DE)**

(72) Erfinder: **Kutscher, Bernhard, Dr.**
**Stresemannstrasse 9**
**W-6457 Maintal 1(DE)**
Erfinder: **Niebch, Georg, Dr.**
**Südring 43**
**W-6458 Rodenbach(DE)**
Erfinder: **Fleischhauer, Ilona, Dr.**
**Zum Mühler 9**
**W-6050 Offenbach(DE)**
Erfinder: **Engel, Jürgen, Prof.**
**Erlenweg 3**
**W-8755 Alzenau(DE)**
Erfinder: **Achterrath-Tuckermann, Ute, Dr.**
**Bahnhofstrasse 120 a**
**W-6457 Maintal 1(DE)**
Erfinder: **Szelenyi, Stefan, Prof.**
**Händelstrasse 32**
**W-8501 Schwaig(DE)**

(54) **(4-Benzylphthalazin-1(2H)-on-2-yl) aminocarbon-säuren mit antiallergischer/antiasthmatischer Wirkung und Verfahren zu deren Herstellung.**

(57) Neue 6-Aminocarbonsäure-Derivate mit antiasthmatischen und antiallergischen Eigenschaften, die zur Herstellung von Arzneimitteln verwendet werden können, werden beschrieben.

EP 0 564 805 A1

Die natürliche proteinogene Aminosäure L-Lysin stellt als 2-Amino-substitutierte 6-Amino-capronsäure eine Verbindung mit vielfältigen physiologischen und biologischen Wirkungen dar. 6-Amino-capronsäure wird großtechnisch für verschiedene Zwecke unter anderem aus ϵ-Caprolactam hergestellt.

Die erfindungsgemäßen 6-Aminocarbonsäure-Derivate sind in 3- oder 4 Position durch benzylsubstituierte Phthalazinonreste substituiert und werden durch folgende Formel beschrieben:

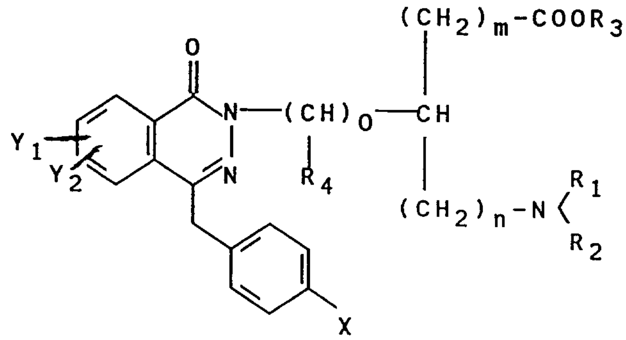

Formel I

In der Verbindung I hat

$Y_1$, $Y_2$ die Bedeutungen Wasserstoff, Halogen, zum Beispiel Fluor oder Chlor, $C_1$-$C_6$-Alkyl, wobei die Kohlenstoffkette gradkettig oder verzweigt sein kann, $C_1$-$C_6$ Alkoxy, $NO_2/NH_2$,

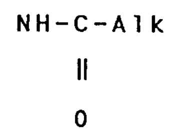

X hat die Bedeutung Wasserstoff, Halogen, zum Beispiel Fluor oder Chlor, $C_1$-$C_6$-Alkyl, wobei die Kohlenstoffkette gradkettig oder verzweigt sein kann, $C_1$-$C_6$ Alkoxy.

$R_1$ und $R_2$ können gleich oder verschieden sein, $R_1$ hat die Bedeutungen Wasserstoff, $C_1$-$C_6$ Alkyl, gradkettig oder verzweigt, Benzyl oder Phenylethyl.

$R_2$ hat die Bedeutungen Wasserstoff, $C_1$-$C_6$ Alkyl, gradkettig oder verzweigt, Benzyl oder Phenylethyl.

$R_1$ und $R_2$ können auch zu einem carbocyclischen Ring mit 5-7 Ringgliedern geschlossen sein.

$R_3$ kann Wasserstoff, eine $C_1$-$C_6$-Alkylgruppe, wobei die Kohlenstoffkette gradkettig oder verzweigt sein kann, oder eine Benzylgruppe, darstellen.

Im Falle von (m + n) = 4 handelt es sich um neue Aminocapronsäure-Derivate.

$R_4$ kann Wasserstoff oder $C_1$-$C_6$-Alkyl, wobei die Kohlenstoffkette gradkettig oder verzweigt sein kann, sein.

m kann die Werte von 0 - 4 annehmen,

n kann die Werte von 0 - 4 annehmen,

O kann die ebenfalls die Werte von 0 - 4 annehmen,

Die erfindungsgemäßen Verbindungen zeichnen sich durch gute antiallergische und antiasthmatische Wirkung aus. So betragt die Hemmwirkung ($ID_{50}$-Werte) im allergisch induzierten Bronchospasmus am Meerschweinchen für die Verbindung gemäß Beispiel 3 0,0099 mg/kg und für die Verbindung gemaß Beispiel 6 0.0008 mg/kg.

Als Testmodell diente eine Modifikation des von Konzett und Rößler (1) beschriebenen Bronchospasmolyseversuchs.

Die Tiere wurden mit Urethan (1.5 g/kg i.p.) narkotisiert. Über einen Hautschnitt wurden Trachea und V. jugularis freigelegt. Für die intravenöse Applikation von Propanolol, Ovalbumin und Testsubstanzen wurde eine Tracheakanüle (Degussa-Eigenbau) eingebunden, die gleichzeitig künstliche Beatmung (Starling Pumpe, Braun Melsungen) und Messung des intratrachealen Druckes (Druckaufnehmer: Venous Pressure Transducer, Statham, Typ W 101 angeschlossen an Brückenverstärker, Heilige und Multicorder 6-Kanal Tintenschreiber, Watanabe, Typ: WA 621-1) ermöglichte. Die gesamte Präparation war nach ca. 15 Minuten beendet. Während des anschließenden Versuches wurden die Tiere mit 40 Atemzügen/Minute und einem

Volumen von etwa 1 ml Raumluft/100 g Körpergewicht beatmet. Der Intratrachealdruck stellte sich dadurch auf 10-15 cm Wassersäule ein. Nach einer Eingewöhnungsphase von ca. 30 Minuten wurden die Testsubstanzen intravenös verabreicht. 10 Minuten später wurde Propanolol verabreicht. 15 Minuten nach der intravenösen Applikation der Testsubstanzen wurde durch Injektion von Ovalbumin eine Bronchokonstriktion ausgelöst. Die dabei auftretende Zunahme des intratrachealen Druckes wurde auf dem Schreiber wenigstens 15 Minuten lang registriert.
Im Modell der Histamin-präkontrahierten Trachea (nach R. W. Foster, J. Pharm. Pharmacol. 12, 189, 1960) beträgt der $IC_{50}$-Wert für die Verbindung gemäß Beispiel 6 35,5 µMol/l und für die Verbindung gemäß Beispiel 3 3,86 µMol/l.

Die Erfindung betrifft auch ein Verfahren zur Herstellung der erfindungsgemäßen Verbindungen. Das Verfahren besteht darin, entsprechend funktionalisierte (in 3- oder in 4-Position) Aminocapronsäurederivate (Formel III) oder deren geschützte oder cyclisierte Derivate aufzubauen und mit aktivierten 4-Benzyl-Phthalazinonen (Formel II) umzusetzen.

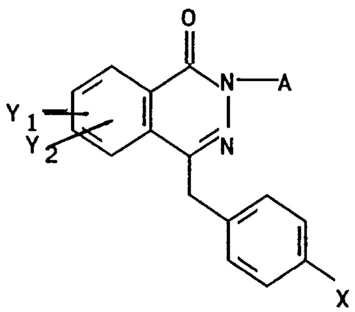

Formel II

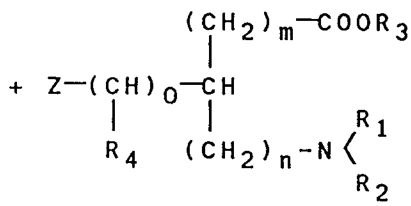

Formel III

X, Y1, und Y2 haben die oben angeführten Bedeutungen.
A hat die Bedeutung Wasserstoff.
$R_1$, $R_2$ und $R_3$ haben die oben angeführten Bedeutungen.
Z kann die Bedeutung Hydroxy, Halogen, Mesylat oder Tosylat haben.
Als Lösungsmittel kommen polare aprotische Lösungsmittel, wie zum Beispiel Dimethylformamid und Dimethylacetamid, weiter aliphatische und aromatische Kohlenwasserstoffe wie zum Beispiel Toluol sowie Gemische aus den vorstehend erwähnten Lösungsmitteln in Frage.

Die Reaktionstemperatur liegt zwischen -40°C und 90°C, insbesondere zwischen -5°C und 50°C.

Als Protonenakzeptoren können Metallhydride, wie zum Beispiel Natriumhydrid oder Alkoholate wie zum Beispiel Natriumethylat oder Natriummethylat dienen.

Beispiel 1:

4-(4-Chlorbenzyl)-2-[5-(N-methyl-N-benzylamino)-1-carboxy-t-butyl-3-pentyl]-1(2H)-phthalazinon

Unter Eisbadkühlung wurden zu 21.6 g 4-(4-Chlorbenzyl)-1(2H)-phthalazinon in 200 ml Dimethylacetamid (DMA) 3 g Natriumhydrid (80 %ig, in Weißöl) hinzugegeben. Anschließend tropft man 37 g 6-(N-Methyl-N-benzylamino)-4-methylsulfonyloxy-capronsäure-t-butylester in 80 ml DMA unter Kühlung hinzu und rührt 0.5 h weiter. Man läßt langsam - über Nacht -auf Raumtemperatur aufwärmen und erwärmt dann 2 h auf 40 °C-50 °C. Man hydrolysiert durch Zugabe von 200 ml Wasser unter Eiskühlung.
Das sich abscheidende Öl wird abgetrennt und in Dichlormethan gelöst. Nach 2-maligem Extrahieren mit Wasser wird die organische Phase getrocknet und eingeengt. Der Rückstand wird über Kieselgel chromatographiert (Eluens: Dichlormethan/Methanol/Ammoniak 25 %).
Man erhält 38.5 g (gelbes Öl).

Zur Charakterisierung wird das Oxalat in Aceton gebildet.
(Smp.: 151-153 °C.)
$^1$H-NMR ($d_6$-DMSO, 500 MHz): $\delta$ = 1,3 (s, 9H, t-Bu); 1.9-2.2 (m,1H); 2.95 (m, 1H);4.05 (br.s,2H; 4.35 (dd,2H); 5.05(br.s,1H); 7.2-7.4 (m,9H); 7.8-8.05 (m,3H); 8.3 (m,1H); 11,3 (br.s,NH)

Beispiel 2:

4-(4-Chlorbenzyl)-2-(5-methylamino-1-carboxymethyl-3-pentyl)-1(2H)-phthalazinon

37 g 4-(4-Chlorbenzyl)-2-[5-(N-methyl-N-benzylamino) -1-carboxy-t-butyl-3-pentyl]-1(2H)-phthalazinon (Beispiel 1) werden in 200 ml 1.2-Dichlorethan gelöst und mit 1.3 g 1.8-Bisdimethylaminonaphthalin versetzt. Bei 4-8 °C tropft man innerhalb von 20 min 17.7 g Chlorameisensäure-1-chlorethylester zu. Man läßt auf Raumtemperatur aufwärmen und erhitzt anschließend 30 min zum Sieden. Das Lösungsmittel wird am Rotationsverdampfer weitgehend entfernt und der Rückstand mit 100 ml Methanol versetzt. Man erhitzt nochmals 2 h unter Rückfluß, entfernt das Lösungsmittel und nimmt den Rückstand mit Wasser auf. Die wäßrige Phase wird mehrmals mit Dichlormethan extrahiert. Die vereinigten organischen Phasen werden mit verdünntem Ammoniak und Wasser gewaschen, getrocknet und eingeengt. Das Rohprodukt wird durch Chromatographie über Kieselgel (Eluens: Dichlormethan/Methanol/Ammoniak 25%) gereinigt.
Ausbeute: 15.3 g Öl
$^1$H-NMR ($CDCl_3$,500 MHz): $\delta$ = 2,0-2.35 (m,6H); 2.4 (s, 3H, N-$CH_3$); 2.5-2.65 (m, 2H); 3.4 (br.s, 1H, NH); 3.6 (s, 3H,$OCH_3$); 4.3 (s, 2H, $CH_2$-Ph); 5.3 (m,1H); 7.15-7.35 (m,4H); 7.7 (m, 3H); 8.45 (m, 1H)
Durch die Behandlung mit Methanol fand eine Umesterung des t-Butyl-in den Methylester statt.

Beispiel 3:

4-(4-Chlorbenzyl)-2-(5-methylamino-1-carboxy-3-pentyl)1(2H)-phthalazinon

15 g 4-(4-Chlorbenzyl)-2-(5-methylamino-1-carboxymethyl-3-penthyl)-1(2H)-phthalazinon (Beispiel 2) werden in 150 ml Methanol gelöst und mit 21 ml 5m Natronlauge versetzt. Man erwärmt 1.5 h auf 50 ° C, engt die Lösung dann am Rotationsverdampfer ein und versetzt den Rückstand mit 105 ml 1 m HCl (pH 6). Man schüttelt die wäßrige Lösung zunächst mit Diethylether aus, um unpolare Verunreinigungen zu entfernen, und extrahiert sie dann mit Dichlormethan. Die vereinigten Dichlormethan-Phasen werden eingeengt und das Rohprodukt aus Ethanol kristallisiert. Das kristalline Produkt wird mit Ether ausgerührt und im Vakuum (55°C/2Torr) getrocknet.
Ausbeute: 9.42 g
Schmelzpunkt: 132 °C

Beispiel 4:

4-(4-Chlorbenzyl)-2-[5-(N-methyl-N-benzylamino)-1-carboxymethyl-2-pentyl]-1(2H)-phthalazinon

Zu einer Suspension von 1.26 g Natriumhydrid (80 %ig in Weißöl) in 20 ml Dimethylformamid (DMF) werden 10.8 g 4-(4-Chlorbenzyl)-1(2H)-phthalazinon in 90 ml DMF unter Eisbadkühlung zugetropft. Dann gibt man - ebenfalls unter Kühlung - die Lösung von 11.5 g 6-(N-Methyl-N-benzylamino)-3-methylsulfonyloxy-capronsäuremethylester in 70 ml DMF hinzu. Man rührt weiter und läßt die Lösung langsam auf Raumtemperatur aufwärmen. Nach beendeter Reaktion (ca.24 h) zerstört man überschüssiges Natriumhydrid durch tropfenweise Zugabe von Wasser unter Eisbadkühlung. Dann gießt man das Gemisch auf Eiswasser und extrahiert mehrmals mit t-Butylmethylether.
Die vereinigten organischen Phasen werden getrocknet und eingeengt. Das Rohprodukt wird über Kieselgel chromatographiert. (Eluens: Dichlormethan/Methanol/Ammoniak 25 %).

Die Ausbeute beträgt 7.9 g (Öl).
$^1$H-NMR ($CDCl_3$, 500 MHz): $\delta$ = 1,4 (m, 1H; 1.55 (m,1H); 1.8 (m, 1H);2.0 (m, 1H); 2.1 (s,3H,N-$CH_3$); 2.4 (m, 2H, $CH_2C=O$); 2.7 (m, 1 H, N-CH; 3.0 (m,1H, N-CH); 3.4 (m, 2H; N-$CH_2$-Ph); 3.6 (s,3H, $OCH_3$); 4.25 (br.s, 2H, $CH_2$Ph); 5.6 (m, 3H); 8.5 (m, 1H)

Beispiel 5:

4-(4-Chlorbenzyl)-2-(5-methylamino-1-carboxymethyl-2-pentyl)-1(2H)-phthalazinon

Die Reaktion wird ausgehend von 6,9 g 4-(4-Chlorbenzyl)2-[5(N-methyl-N-benzylamino)-1-carboxy-methyl-2-pentyl]-1(2H)-phthalazinon (Beispiel 4) wie in Beispiel 2 beschrieben durchgeführt.
Ausbeute: 4,4 g

Beispiel 6:

4-(4-Chlorbenzyl)-2-[5-methylamino-1-carboxy-t-2-pentyl]-1(2H)-phthalazinon

Die Verbindung wird ausgehend von 4,3 g 4-(4-Chlorbenzyl)-2-(5-methylamino-1-carboxymethyl-2-pentyl]-1(2H)-phthalazinon (Beispiel 5) durch alkalische Hydrolyse mit verdünnter NaOH -analog zu Beispiel 3 -dargestellt.
Ausbeute: 3.0 g
Schmelzpunkt: 186-189 °C

Beispiel 7:

4-(4-Fluorbenzyl)-2-[5-(N-benzyl-N-phenylethylamino)-1-(carboxy-2-propyl) -3-pentyl]-1(2H)-phthalazinon

Unter Eisbadkühlung werden zu einer Lösung von 15.7 g 6-(N-Benzyl-N-phenylethylamino)-4-hydroxycapronsäure-2-propylester und 5.0 g Triethylamin in 80 ml Dichlormethan 5.7 g Methansulfonsäurechlorid in 20 ml Dichlormethan langsam zugetropft. Man rührt noch ca. 1,5 h im Eisbad weiter, saugt ausgefallenes Triethylammoniumchlorid ab und wäscht die Lösung zweimal mit gesättigter Natriumhydrogencarbonatlösung sowie einmal mit gesättigter Kochsalzlösung. Die organische Phase wird getrocknet und eingeengt. Das Mesylat fällt als öliger Rückstand an, der ohne weitere Reinigung mit 40 ml Dimethylacetamid (DMA) verdünnt wird und zu der auf ca.5 °C abgekühlten Lösung von 5.6 g 4-(4-Fluorbenzyl)-1(2H)-phthalazinon und 0.75 g Natriumhydrid (80%ig, in Weißöl) in 80 ml DMA getropft wird. Man läßt das Reaktionsgemisch auf Raumtemperatur aufwarmen und rührt noch 5 h bei 50-60 °C nach.

Zug Hydrolyse läßt man unter Eisbadkühlung langsam Wasser zutropfen und extrahiert dann dreimal mit Dichlormethan. Die vereinigten organischen Phasen werden getrocknet und eingeengt. Das zurückbleibende Öl wird über Kieselgel chromatographiert (Eluens: Dichlormethan/Methanol/Ammoniak 25%).
Ausbeute: 10.8 g

Beispiel 8:

4-(4-Fluorbenzyl)-2-[5-(N-phenylethylamino)-1-(carboxy -2-propyl)-3-pentyl]-1(2H)-phthalazinon

10,5g 4-(4-Fluorbenzyl)-2-[5-(N-benzyl-N-phenylethylamino)-1-carboxy-2-propyl-3-pentyl]-1(2H)-phthalazinon (Beispiel 7) werden analog zu Beispiel 2 in 100 ml 1,2-Dichlorethan mit 6.86 1.8-Bisdimethylaminonaphthalin und 4.58 g Chlorameisensäure-1-chlorethylester umgesetzt. Die Aufarbeitung erfolgt wie in Beispiel 2 beschrieben. Das Rohprodukt wird zweimal chromatographiert.
Ausbeute: 1.9 g

Beispiel 9:

4-(4-Fluorbenzyl)-2-[5-(N-phenylethylamino)-1-carboxy-3-pentyl]-1(2 H)-phthalazinon

1.7 g des 2-Propylester aus Beispiel 8 werden mit verdünnter Natronlauge in Analogie zu Beispiel 3 hydrolysiert. Nach dem Ansäuern mit wäßriger HCl (pH 5-6) und Trocknen erhält man 1.0 g des Produktes.
Schmelzpunkt: 190-191 °C

**Patentansprüche**

**1.** Verbindungen der allgemeinen Formel I,

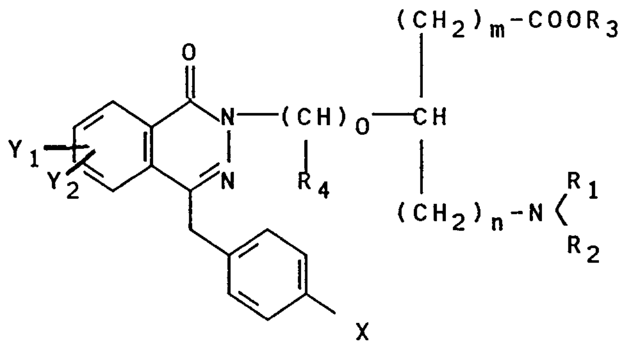

Formel I

wobei $R_1$ Wasserstoff, einen gradkettigen oder verzweigten Alkylrest mit 1 - 6 Kohlenstoffatomen, Benzyl oder Phenylethyl bedeuten,
$R_2$ Wasserstoff, einen gradkettigen oder verzweigten Alkylrest mit 1 - 6 Kohlenstoffatomen, Benzyl oder Phenylethyl bedeuten,
$R_3$ Wasserstoff, einen gradkettigen oder verzweigten Alkylrest mit 1 - 6 Kohlenstoffatomen oder Benzyl bedeuten, $R_1$ und $R_2$ können gleich oder verschieden sein,
X die Bedeutungen Wasserstoff, Halogen, $C_1$-$C_6$-Alkyl oder Alkoxy hat, wobei die Alkylkette eine Länge von 1-6 Kohlenstoffatomen hat,
$Y_1$ und $Y_2$ können gleich oder verschieden sein und die Bedeutungen Wasserstoff, Halogen, Alkyl mit 1-6 Kohlenstoffatomen oder Alkoxy mit 1 - 6 Kohlenstoffatomen haben, wobei
m, n und o die Werte von 0-4 annehmen können.

**2.** Verbindungen gemäß Anspruch 1,
dadurch gekennzeichnet,
daß $R_1$, $R_2$ und $R_3$ Wasserstoff, Alkyl mit 1 - 6 Kohlenstoffatomen, Benzyl oder Phenylethyl bedeuten,
X Chlor oder Fluor bedeuten und
$Y_1$ und $Y_2$ Wasserstoff bedeuten

**3.** Verbindungen der allgemeinen Formel II,
dadurch gekennzeichnet,
daß X die Bedeutung Chlor oder Fluor hat und $Y_1$ und $Y_2$ Wasserstoff bedeuten,
X die Bedeutungen Wasserstoff, Halogen, Hydroxy oder Alkoxy hat, wobei die Alkylkette eine Länge von 1-6 Kohlenstoffatomen hat, und
Y die Bedeutung Halogen, Alkylkette mit 1 - 6 Kohlenstoffatomen oder Alkoxy mit 1 - 6 Kohlenstoffatomen hat und
m, n und o die Werte zwischen 0 und 4 annehmen können.

**4.** Verbindungen der allgemeinen Formel III,
dadurch gekennzeichnet,
daß Z die Bedeutungen Hydroxy, Halogen, Mesylat oder Tosylat hat,
$R_1$ und $R_2$ gleich oder verschieden sein können,
$R_1$, $R_2$ und $R_3$ Wasserstoff, einen gradkettigen oder verzweigten Alkylrest mit 1 - 6 Kohlenstoffatomen, oder Benzyl bedeuten.

**5.** Verfahren zur Herstellung der Verbindungen gemäß Anspruch 1,
dadurch gekennzeichnet,
daß man Verbindungen der Formel II mit Verbindungen der Formel III zu Verbindungen der Formel I umsetzt, wobei Z in der Formel III die Bedeutungen Hydroxy, Halogen, Mesylat oder Tosylat und A die Bedeutung Wasserstoff hat.

**6.** Verbindungen gemaß Anspruch 1,
dadurch gekennzeichnet,
daß man Verbindungen der Formel II mit Verbindungen der Formel III bei -5 °C bis 50 °C in aprotischen Lösungsmitteln oder Kohlenwasserstoffen oder in Gemischen aus aprotischen Lösungsmitteln und Kohlenwasserstoffen in Gegenwart von Protonenakzeptoren umsetzt.

**7.** Arzneimittel,
dadurch gekennzeichnet,
daß sie als Wirkstoff eine oder mehrere Verbindungen gemäß Anspruch 1 oder deren pharmazeutisch verträgliche Salze, gegebenenfalls mit den üblichen pharmazeutischen Träger- und Hilfsstoffen, enthalten.

**8.** Verwendung eines Arzneimittels nach Anspruch 7 zur Behandlung von Allergien, Entzündungen und Asthma.

Europäisches Patentamt

# EUROPÄISCHER RECHERCHENBERICHT

Nummer der Anmeldung

EP 93 10 3179

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 174 464 (ASTA-WERKE AG)<br>19. März 1986<br>* Ansprüche 1-6 *<br>--- | 1-8 | C07D237/32<br>A61K35/50 |
| Y | EP-A-0 222 191 (ASTA-WERKE AG)<br>20. Mai 1987<br>* Ansprüche 1-8 *<br>--- | 1-8 | |
| Y | EP-A-0 289 881 (ASTA PHARMA AG)<br>9. November 1988<br>* Ansprüche 1-6 *<br>--- | 1-8 | |
| Y | EP-A-0 289 939 (ASTA PHARMA AG)<br>9. November 1988<br>* Ansprüche 1-6 *<br>--- | 1-8 | |
| A | EP-A-0 164 593 (PENNWALT CORPORATION)<br>18. Dezember 1985<br>* Ansprüche 1-27 *<br>----- | 1-8 | |
| | | | **RECHERCHIERTE SACHGEBIETE (Int. Cl.5 )** |
| | | | C07D<br>A61K |

Der vorliegende Recherchenbericht wurde für alle Patentansprüche erstellt

| Recherchenort | Abschlußdatum der Recherche | Prüfer |
|---|---|---|
| MUENCHEN | 13 APRIL 1993 | HERZ C.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

1